# EUROPEAN PATENT SPECIFICATION

(11) **EP 0 874 806 B1**
(45) Date of publication and mention of the grant of the patent: **31.10.2001**
(21) Application number: 96942936.4
(22) Date of filing: 19.12.1996
(51) Int. Cl.: C07C 241/04

(54) **PROCESS FOR PREPARATION OF HYDRAZIDES**
VERFAHREN ZUR HERSTELLUNG VON HYDRAZIDEN
PROCEDE DE PREPARATION D'HYDRAZIDES

(30) Priority: 20.12.1995 US 575561
(43) Date of publication of application: 04.11.1998
(73) Proprietor: SCHERING CORPORATION, Kenilworth New Jersey 07033 (US)
(72) Inventor: LEONG, William, Westfield, NJ 07090 (US); SMITH, Lyman, Yonkers, New York 10703 (US)
(74) Representative: von Kreisler, Alek, Dipl.-Chem.
(86) International application number: US9619602
(87) International publication number: WO9722579

(56) References cited:
- WO-A-95/17407
- JOURNAL OF ORGANIC CHEMISTRY, vol. 57, no. 17, 1992, EASTON US, pages 4563-4565, XP000645440 A. ALEXAKIS ET AL.: "Reactivity and diastereosilectivity of Grignard reagents toward the hydrazone functionality in toluene solvent" cited in the application

## Description

Alexakis et al., "Reactivity and Diastereoselectivity of Grignard Reagents toward the Hydrazone Functionality in Tolune Solvent", The Journal of Organic Chemistry, Volume 57, Number 17, pages 4563-4565 (August 14, 1992) disclose that Grignard reagents, in toluene, display a strongly increased reactivity toward the hydrazone functionality. The results of a study of the reaction: 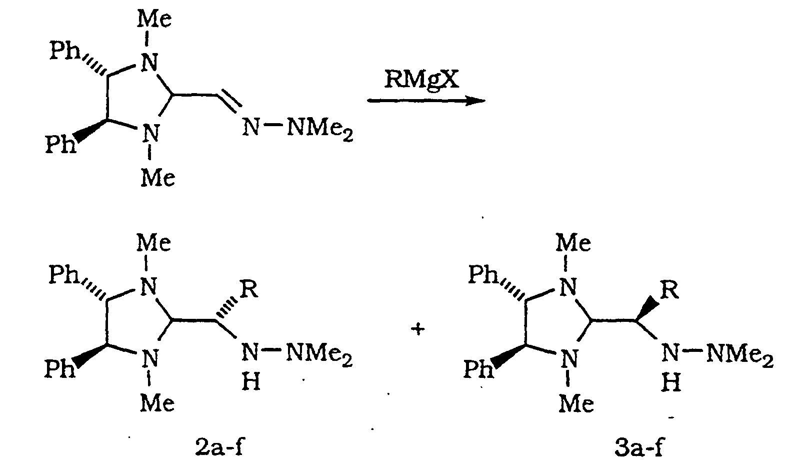 with various Grignard reagents in toluene is disclosed. However, the reactions involve the use of chiral auxiliaries to make enantiomers from dialkyl substituted hydrazones using an excess of Grignard reagent.

WO 95/17407, published June 29. 1995, discloses antifungal compounds whose partial formula is: 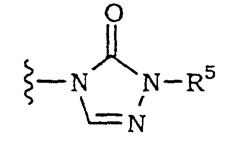 wherein R⁵ can be, amongst others. 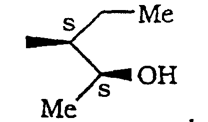 Scheme VI on page 35 describes a preparation of the antifungal compounds. In the reaction sequence an aldehyde (38) is reacted with H₂NNHCHO in methanol to form the hydrazone (39). The hydrazone (39) is reacted with a Grignard reagent, e.g., ethylmagnesium bromide, in dry ether at a temperature of -10°C to room temperature to 24 hours to give the hydrazide (40) wherein the ratio of the S,S isomer: S,R isomer was 94:6. When the Grignard reaction is done in the presence of 1.2 equivalents of bis(trimethylsilyl)acetamide the SS to SR ratio was 99:1. The compounds referred to are disclosed in Scheme VI on page 27. It is believed that the N-NHCHO substituent in hydrazide (40) should be depicted with a single bond to the chiral center.

A process for producing diasteromers in high yield that uses less Grignard reagent, and tolerates the solvent that the Grignard reagent was prepared in (i.e., the process is not adversely effected by the solvent that the Grignard reagent was prepared in) would be a welcome contribution to the art. The invention described herein provides just such a contribution.

### SUMMARY OF THE INVENTION

This invention provides a process for preparing a high diasteromeric yield of high purity of a hydrazide from a hydrazone. The hydrazides are useful as intermediates to antifungal compounds.

In the process, a hydrazone, preferably a hydrazone whose carbonyl group is protected, is reacted with a mixture of Grignard reagents to provide high yields of a specific diasteromer of the corresponding hydrazide. The mixture of Grignard reagents comprises a first Grignard reagent that will add the desired group to the substrate and a second Grignard reagent that is more sterically hindered (i.e., bulkier) than the first Grignard reagent.

Thus, this invention is directed to a process for producing a hydrazide of the formula: 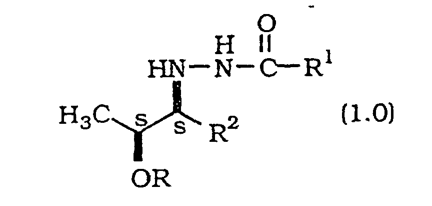 comprising reacting a hydrazone of the formula 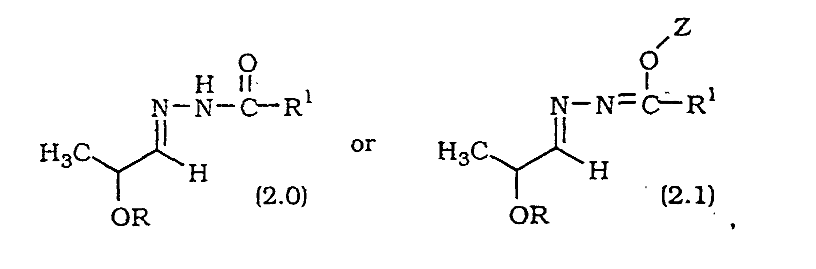 wherein said hydrazone is in toluene, with a mixture of Grignard reagents, wherein said Grignard reagents are in a suitable organic solvent; wherein
(A) Z is a suitable carbonyl protecting group;
(B) R is a suitable -OH protecting group;
(C) R¹ is selected from
   (1) H;
   (2) a non-enolizable alkyl;
   (3) a non-enolizable alkyl group having 1 to 3 substituents selected from halo, C₁-C₆ alkoxy, aryl or aryloxy;
   (4) aryl;
   (5) an aryl group having 1 to 3 substituents selected from halo, alkyl, or C1-C6 alkoxy;
   (6) -S-aryl;
   (7) -S-aryl group having 1 to 3 substituents selected from halo, alkyl, or C₁-C₆ alkoxy;
   (8) -S-alkyl;
   (9) -S-alkyl group having 1 to 3 substituents selected from halo, C₁-C₆ alkoxy, aryl or aryloxy;
   (10) alkoxy;
   (11) an alkoxy group having 1 to 3 substituents selected from halo, C₁-C₆ alkoxy, aryl or aryloxy;
   (12) aryloxy; or
   (13) an aryloxy group having 1 to 3 substituents selected from halo, alkyl, or C₁-C₆ alkoxy;
(D) said mixture of Grignard reagents comprises R²MgX in admixture with R³mgX;
(E) R² is a suitable alkyl, an alkyl group having 1 to 3 substituents selected from halo, C₁-C₆ alkoxy, aryl or aryloxy, alkenyl, alkynyl, aryl, an aryl group having 1 to 3 substituents selected from halo, alkyl, or C₁-C₆ alkoxy, or aralkyl group capable of adding to the -C=N group of the hydrazone to produce the hydrazide;
(F) R³ is a suitable alkyl, an alkyl group having 1 to 3 substituents selected from halo, C₁-C₆ alkoxy, aryl or aryloxy, aryl or an aryl group having 1 to 3 substituents selected from halo, alkyl, or C₁-C₆ alkoxy that is more sterically hindered than said R² group;
(G) X is independently selected from Cl, Br or I for each Grignard reagent;
(H) when said hydrazone is a compound of Formula 2.0 then the reaction is conducted at a temperature of +30 to -40 °C; and
(I) when said hydrazone is a compound of Formula 2.1 then the reaction is conducted at a temperature of +40 to -20 °C.

### DETAILED DESCRIPTION OF THE INVENTION

As used herein, the following terms have the following meanings, unless defined otherwise:
alkenyl - represents straight and branched carbon chains having at least one carbon to carbon double bond and containing from 2 to 12 carbon atoms, preferably from 2 to 6 carbon atoms;
alkyl - (including the alkyl portion of alkoxy and aralkyl) represents straight or branched carbon chains having from 1 to 20 carbons and preferably from 1 to 6 carbons;
alkynyl - represents straight and branched carbon chains having at least one carbon to carbon triple bond and containing from 2 to 12 carbon atoms, preferably from 2 to 6 carbon atoms;
aralkyl - represents an aryl group (as defined below) bound to an alkyl group (as defined above) such as benzyl;
aryl - (including the aryl portion of aryloxy and aralkyl) represents a carbocyclic aromatic group containing from 6 to 15 carbon atoms and having at least one aromatic ring, such as phenyl or naphthyl, with all substitutable carbons of the carbocyclic group being optionally substituted with one or more groups selected from halo, alkyl, hydroxy, alkoxy, phenoxy, -CF₃, amino. alkylamino, dialkylamino or NO₂, for example said aryl group is optionally substituted with 1 to 3 of the above mentioned groups; those skilled in the art will appreciate that only halo substituents which do not interfer with the formation of the Grignard reagents are selected for the substitued aryl R² groups;
BOM - represents benzyloxymethyl;
Bu^{t} or t-Bu - represents tertiary butyl (-C(CH₃)₃);
carbonyl (-C=O) protecting group - represents a protecting group which blocks a -C=O group by binding to the oxygen atom to produce a -C-O-Z group, thereby preventing reactions involving the -C=O group from occurring during the process of the invention: carbonyl protecting groups are well known in the art and methods for the formation and removal of carbonyl protecting groups are also well known, such as those described in Greene, et al., "Protective Groups in Organic Synthesis", 2nd ed., pages 175 to 223, John Wiley & Sons (New York 1991);
EtOAc - represents ethyl acetate;
halo - represents a fluoro, chloro, bromo or iodo group;
hydroxyl or hydroxy (-OH) protecting group - represents a protecting group which blocks an -OH group thereby preventing reactions involving the -OH group from occurring during the process of the invention; hydroxyl protecting groups are well known in the art and methods for the formation and removal of hydroxyl protecting groups are also well known, such as those described in Greene, *et al*., "Protective Groups in Organic Synthesis", 2nd ed., pages 10-144, John Wiley & Sons (New York 1991);
MOM - represents methoxymethyl;
non-enolizable alkyl or substituted alkyl - represents an alkyl group or substituted alkyl group that does not have an acidic hydrogen on the carbon bound to the carbonyl carbon of the hydrazone thereby preventing enolization;
Red-Al - represents sodium bis(2-methoxyethoxy)aluminum hydride;
substituted alkyl - represents an alkyl group having 1 to 3 substituents selected from halo, C₁-C₆ alkoxy, aryl or aryloxy;
substituted aryl - represents an aryl group having 1 to 3 substituents selected from halo, alkyl, or C₁-C₆ alkoxy;
TBME - represents tert-butyl methyl ether;
TBDMS - renresents tert-butyl dimethvlsilvl-i.e.. 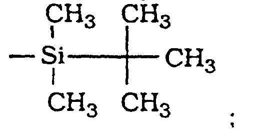
TMS - represents trimethylsilyl;
THF - represents tetrahydrofuran; and
THP - represents tetrahydropyranyl.

The reaction of the hydrazone (2.0 or 2.1) with the mixture of Grignard reagents is preferably done under an inert atmosphere, such as nitrogen. Preferably, hydrazone 2.1 is used. The hydrazone is in an amount of toluene that effectively allows the admixture of the reactants.

Suitable organic solvents for the Grignard reagents are selected from toluene, THF, diethyl ether, TBME or mixtures thereof. Preferably, THF, diethyl ether or TBME is used.

Suitable carbonyl protecting groups (Z) include but are not limited to: C₁ to C₆ alkyl (e.g., methyl or ethyl), trimethylsilyl, triethylsilyl, triisopropylsilyl, dimethylthexylsilyl, acyl (CH₃C(O)-), and -OP(OR⁴)₂ wherein each R⁴ is the same alkyl group (e.g. ethyl), or each R⁴ is the same aryl group (e.g., phenyl).

The reaction of the hydrazone of Formula 2.0 or 2.1 with the mixture of Grignard reagents is conducted at a temperature which allows the reaction to proceed at a reasonable rate without the formation of unwanted by-products. Hydrazones whose carbonyl groups are protected, i.e., compounds of Formula 2.1, can be reacted with the mixture of Grignard reagents at a higher temperature than the unprotected hydrazones of Formula 2.0. Those skilled in the art will appreciate that usually the hydrazone solution is cooled to a low temperature before the the mixture of Grignard reagents is added to the solution. After addition, the resulting reaction mixture is allowed to react at a temperature that is usually higher than the addition temperature of the Grignard reagents.

When the carbonyl group of the hydrazone is unprotected, i.e., a compound of Formula 2.0, the reaction temperature is +30 to -40°C, with 0 to -15°C being preferred, and 0 to -5°C being most preferred. Usually, the hydrazone solution is cooled to the lower end of the temperature range, e.g., -20°C, in preparation for the addition of the Grignard reagents, the temperature is maintained at a higher temperature, e.g., -5°C, during the addition of the Grignard reagents, and then the reaction is allowed to proceed at a higher temperature, e.g., 0°C, to completion.

When a protected hydrazone is used, i.e., a compound of formula 2.1, the reaction temperature is +40 to -20°C, with O to +25 °C being preferred, 10 to 25°C being more preferred, and 25°C being most preferred. Usually, the hydrazone solution is cooled to the lower end of the temperature range, e.g., 0°C, in preparation for the addition of the Grignard reagents, the temperature is maintained at a higher temperature, e.g., below +5°C, during the addition of the Grignard reagents, and then the reaction is allowed to proceed at a higher temperature, e.g., room temperature, to completion.

Representative hydroxyl protecting groups, i.e., substitutent R, include but are not limited to C₁ to C₈ alkyl, phenyl (-C₆H₅), benzyl (-CH₂C₆H₅). allyl, BOM, MOM, TMS, TBDMS, and THP. Preferably, benzyl is used.

Preferably, R¹ is H.

Suitable non-enolizable groups for R¹ include but are not limited to: (1) -C(Cl)₂-alkyl; and (2) C₃ to C₈ secondary alkyl groups such as -CH(CH₃)CH₂CH₃ (s-C₄H₉) or -CH(CH₃)₂; (2) C₃ to C₈ tertiary alkyl groups such as -C(CH₃)₂CH₂CH₃, t-C₄H₉, -C(C₆H₅)(CH₃)₂ and -C(C₆H₅)₃.

Representative examples of R¹ also -OC(CH₃)₃, -OCH₂C₆H₅ (benzyloxy), phenoxy, S-CH₃, S-C₂H₅, and -SC₆H₅. Of these groups -OC(CH₃)₃ is preferred.

The mixture of Grignard reagents comprises R²MgX in admixture with R³MgX. Any group capable of adding to the carbonyl group of an aldehyde or ketone in a Grignard reaction is a suitable R² group for addition to the -C=N- group of the hydrazone. Preferably, R² is a 1°, 2° or 3° alkyl group, more preferably a C₁ to C₈ alkyl group, even more preferably a 1° alkyl group, and most preferably ethyl. Other examples of suitable R² groups include but are not limited to: methyl; (n- or s-)propyl; (n-, s-, or t-)butyl; (n-. s-. or t-)pentyl; (n-, s-, or t-)hexyl; (n-, s-, or t-)heptyl; (n-, s-, or t-)octyl; vinyl; -CH₂CH=CH₂ (allyl); ethynyl; phenyl; and benzyl.

R³ is any suitable group capable of forming a Grignard reagent wherein said group is more sterically hindered than the R² group. Thus, R³ can be selected from the same groups defined for R² provided that the group selected for R³ is more sterically hindered than the R² group. For example, when R² is ethyl then R³ can be selected from: (s- or t-)butyl, (s- or t-)pentyl, (s- or t-)hexyl, or (s- or t-)octyl, with a t-alkyl group being preferred, and t-butyl being most preferred.

X represents a halogen atom selected from Cl, Br or I, with Cl or Br being preferred.

The individual Grignard reagents are known in the art or can be readily made by known techniques.

The R²MgX Grignard reagent is used in a sufficient amount to effectively introduce the R² group into all or substantially all of the substrate (i.e., Formula 2.0 or 2.1). Generally, the R²MgX Grignard reagent is used in an amount of at least 1.0 equivalent (eq) based on the hydrazone 2.0 or 2.1. Usually, for the hydrazone 2.0, the R²MgX Grignard reagent is used in amounts of 1.0 to 4.0 eq, with 2.0 to : 3.0 eq being preferred, and 2.0 to 2.4 eq being most preferred. Usually, for the hydrazone 2.1, the R²MgX Grignard reagent is used in amounts of 1.0 to 1.5 eq, with 1.0 to 1.3 eq being preferred, and 1.1 to 1.2 eq being most preferred.

The R³MgX Grignard reagent is used in a sufficient amount to facilitate the addition of the R² group to produce the desired diasteromer in high yield. The R³MgX Grignard reagent can be used in an excess, relative to the amount of the R²MgX Grignard reagent, which excess is not great enough to cause the addition of the R³ group to the substrate. Generally, the R³MgX Grignard reagent is used in an amount that is at least 0.5 times the amount of the R²MgX Grignard reagent, with at least one times the amount being preferred, 1 to 10 times being more preferred, 1 to 2 times being even more preferred, and one times the amount being most preferred. Thus, it is most preferred that the ratio of R²MgX to R3MgX be 1:1.

The starting reactant of Formula 2.0 or 2.1 can be made according to techniques known in the art. For example, compounds of Formula 2.0, wherein R¹ is H can be made according to the technique disclosed in WO 95/17407 published on June 29, 1995, the disclosure of which is incorporated herein by reference thereto. By using techniques known in the art, compounds of Formula 2.0 wherein R¹ is other than H and/or R is other than benzyl can be made by using the appropriate hydrazone and/or the appropriate -OH protecting group, respectively. Similarily, compounds of Formula 2.1 can be made by selecting the appropriate carbonyl protecting group and the appropriate -OH protecting group.

The benzyloxyamide (5.0) used in the examples can be prepared according to methods known in the art. For example, by the reaction: 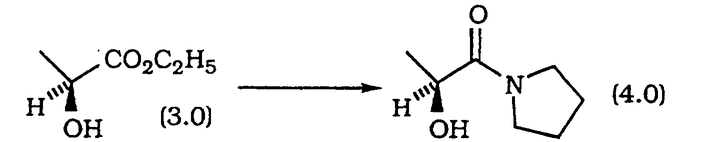 the chiral hydroxy amide (4.0) can be prepared from ethyl (S)-lactate (3.0) via substantially the same procedure as described in Kobayashi et al., Bull. Chem Soc. Jpn., 62, 3038-3040 (1989).

Then, by the reaction: 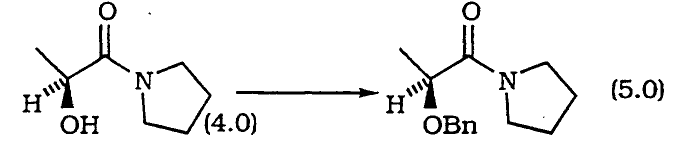 the hydroxy amide (4.0) can be converted to the corresponding benzyl ether (5.0 wherein Bn represents benzyl) via procedures such as the one described in Kobayashi et al., above. Alternatively, benzylation can be carried out via other methods known in the art such as those described in Greene et al., "Protective Groups in Organic Synthesis", 2nd Edition, pages 47-49, John Wiley & Son, New York (1991).

The examples that follow are intended to exemplify the claimed invention, and such examples should not be construed as limiting the disclosure or the claimed invention.

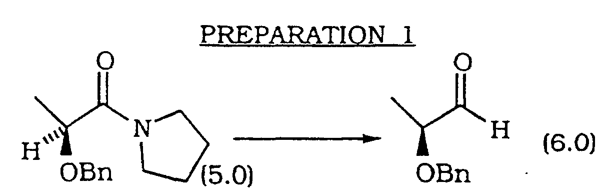

Into a round bottom flask equipped with a mechanical stirrer was placed 5.0 (58.32 g) and toluene (250 mL). The mixture was stirred until 5.0 was dissolved and then cooled to -10 to -5°C. To this solution was dropwise added a solution of Red-Al in toluene (44.1 mL, 3.4M in toluene) over a period of 30 minutes (while maintaining the reaction temperature below -5°C). The reaction was stirred for 8-12 hours at 0°C while monitoring the progress by HPLC. Upon completion, the reaction was quenched with isopropanol (10 mL) at 0°C, stirred for 30 minutes, and then the resulting mixture was poured into 2 N HCl (300mL). The mixture was stirred to ensure dissolution of aluminium salts and the layers were separated. The aqueous phase was extracted with EtOAc (100 mL x3). The combined organic layers were washed successively with water (100 mL), saturaterd aqueous NaHCO₃, brine and dried (MgSO₄). The volatile solvents were removed under vacuo to yield 36.9 g of 6.0 as an oil. MS *m/z* 165 (M+1).

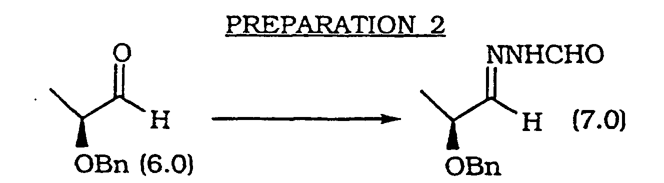

Into a round bottom flask equipped with a mechanical stirrer was placed formic hydrazide (25.23g) and hexane (400 mL) at room temperature. To this suspension was added a solution of aldehyde 6.0 in hexane (65.7g in 200 mL hexane) and the solution was stirred for about 24 hours at room temperature (r.t.). The resulting mixture was partitioned into cold water (300 mL) and EtOAc (100 mL), and the layers were separated. The aqueous phase was extracted with EtOAc (100 mL x4). The combined organic layers were washed with water (100 mL) and dried (MgSO4). The volatile solvents were removed under vacuo to yield 74.91 g of an oil which solidified on standing. The solid was purified by crystallization from minimal 3% TBME in hexane (about 450 mL) and gentle heating. The white crystals thus formed were cooled to 0°C, filtered and dried in a draft vacuum chamber (r.t.) to afford 66.5 g ( 81% yield) of hydrazone 7.0 as a white solid. MS *m/z* 207.0 (M+ 1).

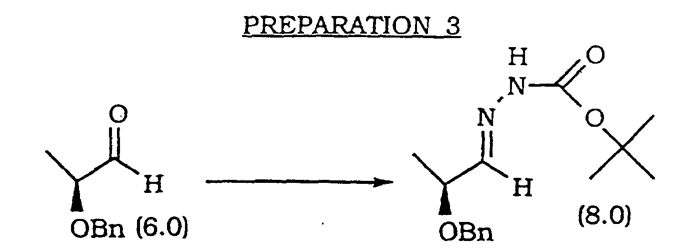

Into a round bottom flask equipped with a mechanical stirrer was placed t-butyl carbazate (1.38g) and hexane (10 mL) at room temperature. To this suspension was added a solution of aldehyde 6.0 in hexane (1.64g in 10 mL hexane) and the solution was stirred for 24 hours at room temperature. The resulting mixture was partitioned into cold water (15 mL) and stirred for 30 minutes at room temperature. The solids that formed were filtered and dried in a draft vacuum chamber (r.t. for 16 hours) to afford 2.49 g (90% yield) of hydrazone 8.0 as a white crystalline needle. 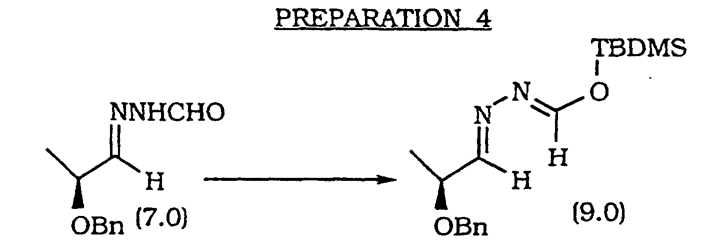 Into a round bottom flask equipped with a mechanical stirrer was placed 7.0 (61.89g) and TBME (600 mL) at room temperature. To this solution at room temperature was added triethylamine (63.0 ml), followed by TBDMS-Cl (49.74g), and the solution was stirred for 24 hours at room temperature. The resulting mixture was filtered through a pad of celite and concentrated in vacuo to an oil. This oil was dissolved in TBME (100 mL) and filtered through a pad of celite. The solution was concentrated in vacuo to afford an oil. The oil thus obtained weighed 91.0 g (95%). Proton NMR showed 97% of silylated hydrazone. MS *m/z* 207.1 ([M-TBDMS]+1). This oil was not purified.

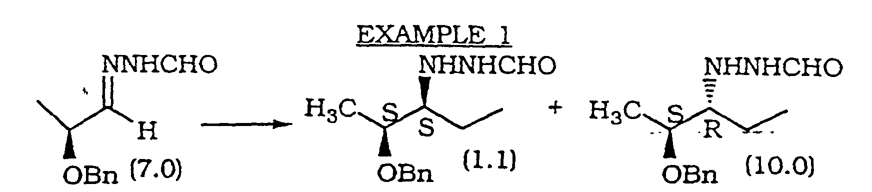

Into a round bottom flask equipped with a stirrer, and under a nitrogen atmosphere, was charged ethylmagnesium chloride (176 mL, 352 mmol, 2.OM in THF) at room temperature (24-28°C). To this was charged t-butylmagnesium chloride (469 mL, 352 mmol, 0.74 M in THF) and the solution was stirred at room temperature. The resulting solution is approximately 0.6 M in "ethyl" concentration.

Into a separate round bottom flask equipped with a stirrer and an addition funnel, and under a nitrogen atmosphere, was placed hydrazone 7.0 (33.0 g) and toluene (480 mL) at room temperature. This solution was cooled to -20°C and was treated to a dropwise addition of the Grignard reagent (the temperature was maintained below -5°C). Upon addition, the reaction mixture was stirred at 0°C for 24 hours. The reaction was monitored for completion by HPLC. The resulting mixture was quenched by pouring into 2 L of ice-water and extracted with TBME (500 ml x 3). The organic layers were washed with saturated aqueous NaCl and dried (MgSO₄). The volatile solvents were removed under vacuo to yield 37.5 g of an oil. HPLC assay indicated the yield to be 63% pure 1.1 with an SS:SR ratio (1.1:10.0) of 97:3. MS *m/z* 259.1 (M+1).

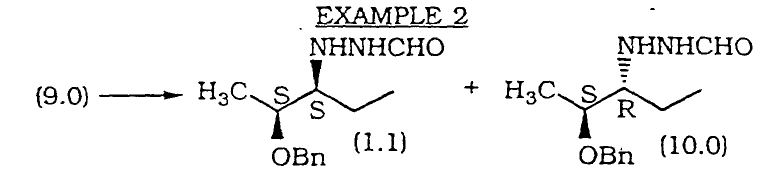

Into a round bottom flask equipped with a stirrer, and under an inert atmosphere was charged ethylmagnesium chloride (157.6 mL, 315 mmol. 2.OM in THF) at room temperature (24-28°C). To this was charged t-butylmagnesium chloride (370 mL, 315 mmol, 0.85 M in THF) and the solution was stirred at room temperature. The resulting solution was approximately 0.597 M in "ethyl" concentration.

Into a separate round bottom flask equipped with a stirrer and an addition funnel, and under a nitrogen atmosphere, was placed TDBMS-hydrazone 9.0 (89.73 g) and toluene (420 mL) at room temperature. This solution was cooled to 0°C and was treated to a dropwise addition of the Grignard reagent (while the temperature was maintained below 5°C). Upon addition the reaction mixture was stirred at room temperature for 24 hours. The reaction was monitored for completion by HPLC. The resulting mixture was quenched by pouring into ice-water and extracted with TBME (800 mL x 3).

The organic solvents were removed under vacuum and the resulting oil was partitioned into heptane (700 mL) and 1N HCl (700 mL). The two phase mixture was vigorously stirred for 30 minutes before the layers were separated and the organic layer washed with 1N HCl. The combined acid layers were neutralized with 6N NaOH to pH 6 and with solid sodium bicarbonate to pH 8. This aqueous layer was extracted with methylene chloride (400 mL x 5) and the combined organic layers were dried (MgSO₄). The volatile solvents were removed under vacuo to yield 48.9 g of an oil. HPLC assay indicated the yield to be 95% pure 1.1 with an SS:SR ratio (1.1:10.0) of 99:1. Proton NMR indicated the oil to be ≥ 95% pure 1.1. MS *m/z* 259.1 (M+ 1).

## Claims

1. A process for producing a hydrazide of the formula: 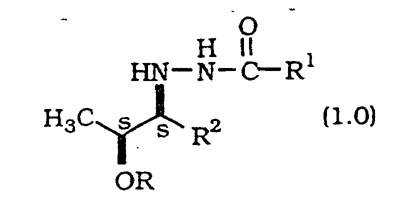 comprising reacting a hydrazone of the formula 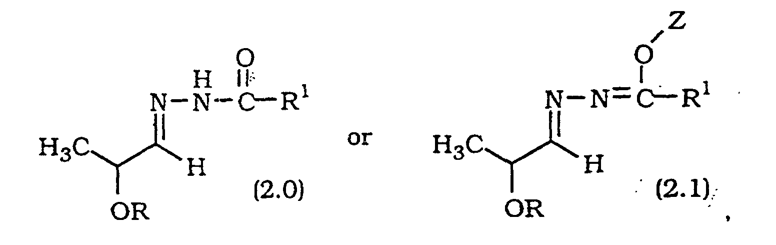 wherein said hydrazone is in toluene, with a mixture of Grignard reagents, wherein said Grignard reagents are in a suitable organic solvent; wherein
(A) Z is a suitable carbonyl protecting group;
(B) R is a suitable -OH protecting group;
(C) R¹ is selected from
(1) H;
(2) a non-enolizable alkyl;
(3) a non-enolizable alkyl group having 1 to 3 substituents selected from halo, C₁-C₆ alkoxy, aryl or aryloxy;
(4) aryl;
(5) an aryl group having 1 to 3 substituents selected from halo, alkyl, or C1-C6 alkoxy;
(6) -S-aryl;
(7) -S-aryl group having 1 to 3 substituents selected from halo, alkyl, or C₁-C₆ alkoxy;
(8) -S-alkyl;
(9) -S-alkyl group having 1 to 3 substituents selected from halo, C₁-C₆ alkoxy, aryl or aryloxy;
(10) alkoxy;
(11) an alkoxy group having 1 to 3 substituents selected from halo, C₁-C₆ alkoxy, aryl or aryloxy;
(12) aryloxy; or
(13) an aryloxy group having 1 to 3 substituents selected from halo, alkyl, or C₁-C₆ alkoxy;
(D) said mixture of Grignard reagents comprises R²MgX in admixture with R³mgX;
(E) R² is a suitable alkyl, an alkyl group having 1 to 3 substituents selected from halo, C₁-C₆ alkoxy, aryl or aryloxy; alkenyl, alkynyl, aryl, an aryl group having 1 to 3 substituents selected from halo, alkyl, or C₁-C₆ alkoxy; or aralkyl group capable of adding to the -C=N group of the hydrazone to produce the hydrazide;
(F) R³ is a suitable alkyl, an alkyl group having 1 to 3 substituents selected from halo, C₁-C₆ alkoxy, aryl or aryloxy; aryl or an aryl group having 1 to 3 substituents selected from halo, alkyl, or C₁-C₆ alkoxy that is more sterically hindered than said R² group;
(G) X is independently selected from Cl, Br or I for each Grignard reagent;
(H) when said hydrazone is a compound of Formula 2.0 then the reaction is conducted at a temperature of +30 to -40 °C; and
(I) when said hydrazone is a compound of Formula 2.1 then the reaction is conducted at a temperature of +40 to -20 °C.

2. The process of claim 1 wherein Z is TBDMS.

3. The process of claim 1 wherein R is benzyl.

4. The process of claim 1 wherein R¹ is H or -OC(CH₃)₃.

5. The process of claim 1 wherein R² is a 1° alkyl group and R³ is a 3° alkyl group, and X is Cl or Br.

6. The process of claim 5 wherein R² is ethyl and R³ is t-butyl, and X is Cl or Br.

7. The process of claim 6 wherein X is Cl.

8. The process of claim 1 wherein said hydrazone is a compound of Formula 2.1 said R²MgX Grignard reagent is used in amounts of 1.0 to 2.0 eq. and said R³MgX Grignard reagent is used in an amount that is 1 to 2 times the amount of said R²MgX Grignard reagent.

9. The process of claim 8 wherein the reaction is conducted at 0 to 25 °C.

10. The process of claim 1 wherein said hydrazone is a compound of Formula 2.0 said R²MgX Grignard reagent is used in amounts of 1.0 to 4.0 eq. and said R³MgX Grignard reagent is used in an amount that is 1 to 2 times the amount of said R²MgX Grignard reagent.

11. The process of claim 10 wherein R is benzyl and R¹ is H or -OC(CH₃)₃.

12. The process of claim 11 wherein the reaction temperature is 0 to -15° C.

13. The process of claim 1 wherein said organic solvent is selected from: toluene or diethyl ether.

14. The process of claim 1 wherein Z is TBDMS; R is benzyl; R¹ is H or-OC(CH₃)₃; R² is a 1° alkyl; R³ is a 3° alkyl; and X is Cl or Br.

15. The process of claim 14 wherein the hydrazone is a compound of Formula 2.1, said R²MgX Gignard reagent is used in amounts of 1.0 to 2.0 eq. and said R³MgX Grignard reagent is used in an amount that is 1 to 2 times the amount of said R²MgX Grignard reagent.

16. The process of claim 15 wherein R¹ is H; R² is ethyl; and R³ is t-butyl.

17. The process of claim 16 wherein said reaction temperature is 0 to 25 °C, and said solvent is toluene.

18. The process of claim 14 wherein the hydrazone is a compound of Formula 2.0 said R²MgX Grignard reagent is used in amounts of 1.0 to 4.0 eq, and said R³MgX Grignard reagent is used in an amount that is 1 to 2 times the amount of said R²MgX Grignard reagent.

19. The process of claim 18 wherein R¹ is -OC(CH₃)₃; R² is ethyl; and R³ is t-butyl.

20. The process of claim 17 wherein said reaction temperature is 0 to -15 °C, and said solvent is toluene.

## Patentansprüche

1. Verfahren zur Herstellung eines Hydrazids der Formel 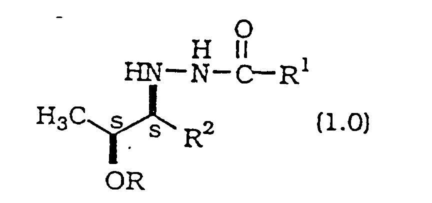 umfassend das Umsetzen eines Hydrazons der Formel 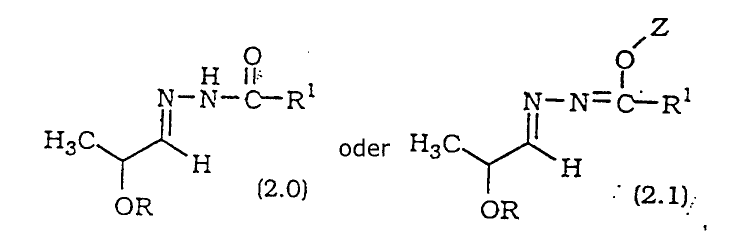 wobei das Hydrazon in Toluol vorliegt, mit einem Gemisch von Grignard-Reagentien, wobei die Grignard-Reagentien in einem geeigneten organischen Lösungsmittel vorliegen; wobei
(A) Z eine geeignete Carbonylschutzgruppe ist;
(B) R eine geeignete OH-Schutzgruppe ist;
(C) R¹ ausgewählt ist aus
(1) H;
(2) einem nichtenolisierbaren Alkyl;
(3) einer nichtenolisierbaren Alkylgruppe mit 1 bis 3 Substituenten, die aus Halogen, C₁-C₆-Alkoxy, Aryl oder Aryloxy ausgewählt sind;
(4) Aryl;
(5) einer Arylgruppe mit 1 bis 3 Substituenten, die aus Halogen, Alkyl oder C₁-C₆-Alkoxy ausgewählt sind;
(6) -S-aryl;
(7) einer -S-arylgruppe mit 1 bis 3 Substituenten, die aus Halogen, Alkyl oder C₁-C₆-Alkoxy ausgewählt sind;
(8) -S-alkyl;
(9) einer -S-alkylgruppe mit 1 bis 3 Substituenten, die aus Halogen, C₁-C₆-Alkoxy, Aryl oder Aryloxy ausgewählt sind;
(10) Alkoxy;
(11) einer Alkoxygruppe mit 1 bis 3 Substituenten, die aus Halogen, C₁-C₆-Alkoxy, Aryl oder Aryloxy ausgewählt sind;
(12) Aryloxy; oder
(13) einer Aryloxygruppe mit 1 bis 3 Substituenten, die aus Halogen, Alkyl oder C₁-C₆-Alkoxy ausgewählt sind;
(D) das Gemisch von Grignard-Reagentien R²MgX im Gemisch mit R³MgX umfasst;
(E) R² eine geeignete Gruppe ist, und zwar Alkyl, eine Alkylgruppe mit 1 bis 3 Substituenten, die aus Halogen, C₁-C₆-Alkoxy, Aryl oder Aryloxy ausgewählt sind; Alkenyl, Alkinyl, Aryl, eine Arylgruppe mit 1 bis 3 Substituenten, die aus Halogen, Alkyl oder C₁-C₆-Alkoxy ausgewählt sind; oder eine Aralkylgruppe; wobei R² an die -C=N-Gruppe des Hydrazons addiert werden kann, so dass das Hydrazid entsteht;
(F) R³ eine geeignete Gruppe ist, und zwar Alkyl, eine Alkylgruppe mit 1 bis 3 Substituenten, die aus Halogen, C₁-C₆-Alkoxy, Aryl oder Aryloxy ausgewählt sind; Aryl oder eine Arylgruppe mit 1 bis 3 Substituenten, die aus Halogen, Alkyl oder C₁-C₆-Alkoxy ausgewählt sind; wobei R³ stärker sterisch gehindert ist als die R²-Gruppe;
(G) X für jedes Grignard-Reagens unabhängig aus Cl, Br oder I ausgewählt ist;
(H) wenn das Hydrazon eine Verbindung der Formel 2.0 ist, die Reaktion bei einer Temperatur von +30 bis -40°C durchgeführt wird; und
(I) wenn das Hydrazon eine Verbindung der Formel 2.1 ist, die Reaktion bei einer Temperatur von +40 bis -20 °C durchgeführt wird.

2. Verfahren gemäß Anspruch 1, wobei Z TBDMS ist.

3. Verfahren gemäß Anspruch 1, wobei R Benzyl ist.

4. Verfahren gemäß Anspruch 1, wobei R¹ H oder -OC(CH₃)₃ ist.

5. Verfahren gemäß Anspruch 1, wobei R² eine primäre Alkylgruppe ist und R³ eine tertiäre Alkylgruppe ist und X Cl oder Br ist.

6. Verfahren gemäß Anspruch 5, wobei R² Ethyl ist und R³ t-Butyl ist und X Cl oder Br ist.

7. Verfahren gemäß Anspruch 6, wobei X Cl ist.

8. Verfahren gemäß Anspruch 1, wobei das Hydrazon eine Verbindung der Formel 2.1 ist, das R²MgX-Grignard-Reagens in Mengen von 1,0 bis 2,0 Äqu. verwendet wird und das R³MgX-Grignard-Reagens in einer Menge verwendet wird, die dem Ein- bis Zweifachen der Menge des R2MgX-Grignard-Reagens entspricht.

9. Verfahren gemäß Anspruch 8, wobei die Reaktion bei 0 bis 25 °C durchgeführt wird.

10. Verfahren gemäß Anspruch 1, wobei das Hydrazon eine Verbindung der Formel 2.0 ist, das R²MgX-Grignard-Reagens in Mengen von 1,0 bis 4,0 Äqu. verwendet wird und das R³MgX-Grignard-Reagens in einer Menge verwendet wird, die dem Ein- bis Zweifachen der Menge des R2MgX-Grignard-Reagens entspricht.

11. Verfahren gemäß Anspruch 10, wobei R Benzyl ist und R¹ H oder -OC(CH₃)₃ ist.

12. Verfahren gemäß Anspruch 11, wobei die Reaktionstemperatur 0 bis -15 °C beträgt.

13. Verfahren gemäß Anspruch 1, wobei das organische Lösungsmittel aus Toluol oder Diethylether ausgewählt ist.

14. Verfahren gemäß Anspruch 1, wobei Z TBDMS ist, R Benzyl ist, R¹ H oder -OC(CH₃)₃ ist, R² eine primäre Alkylgruppe ist, R³ eine tertiäre Alkylgruppe ist und X Cl oder Br ist.

15. Verfahren gemäß Anspruch 14, wobei das Hydrazon eine Verbindung der Formel 2.1 ist, das R²MgX-Grignard-Reagens in Mengen von 1,0 bis 2,0 Äqu. verwendet wird und das R³MgX-Grignard-Reagens in einer Menge verwendet wird, die dem Ein- bis Zweifachen der Menge des R2MgX-Grignard-Reagens entspricht.

16. Verfahren gemäß Anspruch 15, wobei R¹ H ist, R² Ethyl ist und R³ t-Butyl ist.

17. Verfahren gemäß Anspruch 16, wobei die Reaktionstemperatur 0 bis 25 °C beträgt und das Lösungsmittel Toluol ist.

18. Verfahren gemäß Anspruch 14, wobei das Hydrazon eine Verbindung der Formel 2.0 ist, das R²MgX-Grignard-Reagens in Mengen von 1,0 bis 4,0 Äqu. verwendet wird und das R³MgX-Grignard-Reagens in einer Menge verwendet wird, die dem Ein- bis Zweifachen der Menge des R²MgX-Grignard-Reagens entspricht.

19. Verfahren gemäß Anspruch 18, wobei R¹ -OC(CH₃)₃ ist, R² Ethyl ist und R³ t-Butyl ist.

20. Verfahren gemäß Anspruch 17, wobei die Reaktionstemperatur 0 bis -15 °C beträgt und das Lösungsmittel Toluol ist.

## Revendications

1. Procédé de production d'un hydrazide de formule : 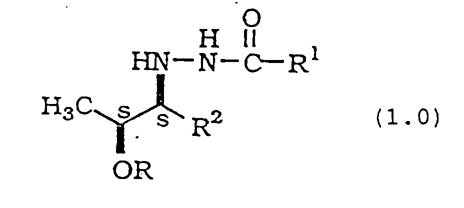 qui comprend la réaction d'une hydrazone de formule : 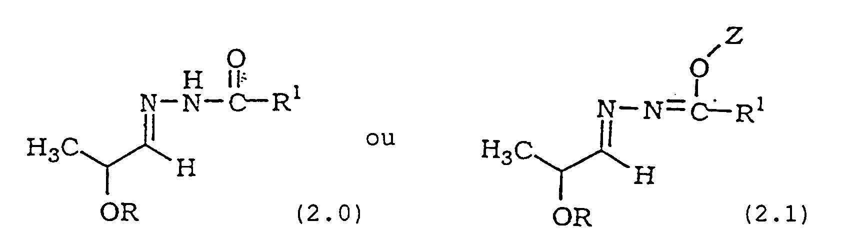 qui est dans du toluène, avec un mélange de réactifs de Grignard qui sont dans un solvant organique approprié, procédé dans lequel :
(A) Z représente un groupe approprié, protégeant le groupe carbonyle,
(B) R représente un groupe approprié, protégeant le groupe -OH,
(C) R¹ est choisi parmi :
(1) H,
(2) les groupes alkyle non-énolisables,
(3) les groupes alkyle non-énolisables, portant 1 à 3 substituants choisis parmi les atomes d'halogène, les groupes alcoxy en C₁ à C₆, les groupes aryle et les groupes aryloxy,
(4) les groupes aryle,
(5) les groupes aryle portant 1 à 3 substituants choisis parmi les atomes d'halogène, les groupes alkyle et les groupes alcoxy en C₁ à C₆,
(6) les groupes -S-aryle,
(7) les groupes -S-aryle portant 1 à 3 substituants choisis parmi les atomes d'halogène, les groupes alkyle et les groupes alcoxy en C₁ à C₆,
(8) les groupes -S-alkyle,
(9) les groupes -S-alkyle portant 1 à 3 substituants choisis parmi les atomes d'halogène, les groupes alcoxy en C₁ à C₆, les groupes aryle et les groupes aryloxy,
(10) les groupes alcoxy,
(11) les groupes alcoxy portant 1 à 3 substituants choisis parmi les atomes d'halogène, les groupes alcoxy en C₁ à C₆, les groupes aryle et les groupes aryloxy,
(12) les groupes aryloxy, et
(13) les groupes aryloxy portant 1 à 3 substituants choisis parmi les atomes d'halogène, les groupes alkyle et les groupes alcoxy en C₁ à C₆,
(D) ledit mélange de réactifs de Grignard comprend R²MgX en mélange avec R³MgX,
(E) R² représente un groupe alkyle approprié, un groupe alkyle portant 1 à 3 substituants choisis parmi les atomes d'halogène, les groupes alcoxy en C₁ à C₆, les groupes aryle et les groupes aryloxy, un groupe alcényle, un groupe alcynyle, un groupe aryle, un groupe aryle portant 1 à 3 substituants choisis parmi les atomes d'halogène, les groupes alkyle et les groupes alcoxy en C₁ à C₆, ou un groupe aralkyle, capable de s'additionner sur la double liaison C=N de l'hydrazone pour produire l'hydrazide,
(F) R³ représente un groupe alkyle approprié, un groupe alkyle portant 1 à 3 substituants choisis parmi les atomes d'halogène, les groupes alcoxy en C₁ à C₆, les groupes aryle et les groupes aryloxy, un groupe aryle ou un groupe aryle portant 1 à 3 substituants choisis parmi les atomes d'halogène, les groupes alkyle et les groupes alcoxy en C₁ à C₆, qui est plus encombré stériquement que ledit groupe R²,
(G) X représente indépendamment pour chaque réactif de Grignard un atome de chlore, de brome ou d'iode,
(H) lorsque ladite hydrazone est un composé de formule 2.0, la réaction est conduite à une température comprise dans l'intervalle allant de +30 à -40 °C, et
(I) lorsque ladite hydrazone est un composé de formule 2.1, la réaction est conduite à une température comprise dans l'intervalle allant de +40 à -20 °C.

2. Procédé selon la revendication 1, dans lequel Z représente le groupe TBDMS.

3. Procédé selon la revendication 1, dans lequel R représente le groupe benzyle.

4. Procédé selon la revendication 1, dans lequel R¹ représente H ou le groupe -OC(CH₃)₃.

5. Procédé selon la revendication 1, dans lequel R² représente un groupe alkyle primaire, R³ représente un groupe alkyle tertiaire et X représente Cl ou Br.

6. Procédé selon la revendication 5, dans lequel R² représente le groupe éthyle, R³ représente le groupe t-butyle et X représente Cl ou Br.

7. Procédé selon la revendication 6, dans lequel X représente Cl.

8. Procédé selon la revendication 1, dans lequel ladite hydrazone est un composé de formule 2.1, ledit réactif de Grignard R²MgX est utilisé en une quantité de 1,0 à 2,0 équivalents et ledit réactif de Grignard R³MgX est utilisé en une quantité qui représente 1 à 2 fois la quantité dudit réactif de Grignard R²MgX.

9. Procédé selon la revendication 8, dans lequel la réaction est conduite à une température de 0 à 25 °C.

10. Procédé selon la revendication 1, dans lequel ladite hydrazone est un composé de formule 2.0, ledit réactif de Grignard R²MgX est utilisé en une quantité de 1,0 à 4,0 équivalents et ledit réactif de Grignard R³MgX est utilisé en une quantité qui représente 1 à 2 fois la quantité dudit réactif de Grignard R²MgX.

11. Procédé selon la revendication 10, dans lequel R représente un groupe benzyle et R¹ représente H ou un groupe -OC(CH₃)₃.

12. Procédé selon la revendication 11, dans lequel la température de réaction est de 0 à -15 °C.

13. Procédé selon la revendication 1, dans lequel ledit solvant organique est le toluène ou l'oxyde diéthylique.

14. Procédé selon la revendication 1, dans lequel Z est le groupe TBDMS, R est le groupe benzyle, R¹ est H ou le groupe -OC(CH₃)₃, R² est un groupe alkyle primaire, R³ est un groupe alkyle tertiaire et X représente Cl ou Br.

15. Procédé selon la revendication 14, dans lequel l'hydrazone est un composé de formule 2.1, ledit réactif de Grignard R²MgX est utilisé en une quantité de 1,0 à 2,0 équivalents et ledit réactif de Grignard R³MgX est utilisé en une quantité qui représente 1 à 2 fois la quantité dudit réactif de Griqnard R²MgX.

16. Procédé selon la revendication 15, dans lequel R¹ représente H, R² représente un groupe éthyle et R³ représente un groupe t-butyle.

17. Procédé selon la revendication 16, dans lequel ladite température de réaction est de 0 à 25 °C et ledit solvant est le toluène.

18. Procédé selon la revendication 14, dans lequel l'hydrazone est un composé de formule 2.0, ledit réactif de Grignard R²MgX est utilisé en une quantité de 1,0 à 4,0 équivalents, et ledit réactif de Grignard R³MgX est utilisé en une quantité qui représente 1 à 2 fois la quantité dudit réactif de Grignard R²MgX.

19. Procédé selon la revendication 18, dans lequel R¹ représente un groupe -OC(CH₃)₃, R² représente un groupe éthyle et R³ représente un groupe t-butyle.

20. Procédé selon la revendication 17, dans lequel ladite température de réaction est de 0 à -15 °C et ledit solvant est le toluène.
